(19) Europäisches Patentamt / European Patent Office / Office européen des brevets

(11) EP 2 306 967 B1

(12) EUROPÄISCHE PATENTSCHRIFT

(45) Veröffentlichungstag und Bekanntmachung des Hinweises auf die Patenterteilung:
**29.07.2015 Patentblatt 2015/31**

(21) Anmeldenummer: **09779667.6**

(22) Anmeldetag: **08.06.2009**

(51) Int Cl.:
*A61K 8/81* (2006.01) *A61Q 5/10* (2006.01)
*D06P 3/14* (2006.01) *A61Q 5/00* (2006.01)

(86) Internationale Anmeldenummer:
**PCT/EP2009/057014**

(87) Internationale Veröffentlichungsnummer:
**WO 2010/015442 (11.02.2010 Gazette 2010/06)**

# (54) COLORATION MIT KATIONISIERBAREN POLYMER

COLORATION USING CATIONIZABLE POLYMER

COLORATION AU MOYEN D'UN POLYMÈRE CATIONISABLE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **06.08.2008 DE 102008036535**

(43) Veröffentlichungstag der Anmeldung:
**13.04.2011 Patentblatt 2011/15**

(73) Patentinhaber: **Henkel AG & Co. KGaA**
**40589 Düsseldorf (DE)**

(72) Erfinder:
- **GOUTSIS, Konstantin**
**41812 Erkelenz (DE)**
- **REICHERT, Anja**
**40629 Düsseldorf (DE)**

(56) Entgegenhaltungen:
**EP-A- 1 707 183** **WO-A-2009/085838**
**FR-A- 2 312 233** **FR-A- 2 769 217**
**FR-A- 2 820 032** **GB-A- 2 000 026**
**JP-A- 2003 342 137**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 306 967 B1

## Beschreibung

Die Erfindung betrifft ein Mittel zur Farbveränderung von keratinhaltigen Fasern, insbesondere menschlichen Haaren, welches in einem kosmetischen Träger mindestens ein Oxidationsfarbstoffvorprodukt und ein kationisierbares Copolymer enthält. Weiterhin betrifft die Erfindung ein Verfahren von solchen Mitteln auf keratinhaltigen Fasern. Darüber hinaus betrifft die Erfindung die Verwendung dieses Mittels zur Verbesserung des Farberhalts der Färbung und zur Verbesserung der Grauabdeckung.

Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepasst werden. Zur modischen Farbgestaltung von Frisuren oder zur Kaschierung von ergrautem oder gar weißem Haar mit modischen oder natürlichen Farbtönen greift der Verbraucher zu farbverändernden Mitteln. Diese Mittel sollen neben der gewünschten Färbeleistung möglichst minimale Schädigungen auf dem Haar hervorrufen und vorzugsweise sogar zusätzliche Pflegeeigenschaften besitzen.

Zur Bereitstellung farbverändernder kosmetischer Mittel, insbesondere für die Haut oder keratinhaltige Fasern wie beispielsweise menschliche Haare, kennt der Fachmann je nach Anforderungen an die Färbung diverse Färbesysteme. Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich zwar durch hervorragende, lang anhaltende Färbeergebnisse aus. Für natürlich wirkende Färbungen muss aber üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet. Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Substrat aufziehen und keinen oxidativen Prozess zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als die oxidativen Färbungen, so dass dann sehr viel schneller eine vielfach unerwünschte Nuancenverschiebung oder gar ein sichtbarer homogener Farbverlust eintritt. Schließlich hat ein weiteres Färbeverfahren große Beachtung gefunden. Bei diesem Verfahren werden Vorstufen des natürlichen Haarfarbstoffes Melanin auf das Substrat, z. B. Haare, aufgebracht; diese bilden dann im Rahmen oxidativer Prozesse im Haar naturanaloge Farbstoffe aus. Die Ausfärbung kann dabei mit Luftsauerstoff als einzigem Oxidationsmittel erfolgen, so dass auf keine weiteren Oxidationsmittel zurückgegriffen werden muss. Eine weitere Möglichkeit zur Farbveränderung bietet die Verwendung von Färbemitteln, welche sogenannte Oxofarbstoffvorprodukte enthalten. Eine erste Klasse der Oxofarbstoffvorprodukte sind Verbindungen mit mindestens einer reaktiven Carbonyl-Gruppe. Eine zweite Klasse der Oxofarbstoffvorprodukte bilden CH-acide Verbindungen und Verbindungen mit primärer oder sekundärer Aminogruppe oder Hydroxygruppe, die wiederum ausgewählt werden aus Verbindungen der Gruppe, die gebildet wird aus primären oder sekundären aromatischen Aminen, stickstoffhaltigen heterozyklischen Verbindungen sowie aromatischen Hydroxyverbindungen. Die vorgenannten Komponenten sind im Allgemeinen selbst keine Farbstoffe, und eignen sich daher jede für sich genommen allein nicht zur Färbung keratinhaltiger Fasern. In Kombination bilden sie in einem nichtoxidativen Prozess der sogenannten Oxofärbung Farbstoffe aus. Die resultierenden Färbungen besitzen teilweise Farbechtheiten auf der keratinhaltigen Faser, die mit denen der Oxidationsfärbung vergleichbar sind. Das mit der schonenden Oxofärbung erzielbare Nuancenspektrum ist sehr breit und die erhaltene Färbung weist oftmals eine akzeptable Brillanz und Farbtiefe auf. Sollen Substrate aufgehellt oder gar gebleicht werden, werden die das Substrat färbenden Farbstoffe meist oxidativ unter Einsatz von entsprechenden Oxidationsmitteln, wie beispielsweise Wasserstoffperoxid, entfärbt.

Um den Pflegezustand der Fasern zu verbessern, ist es seit langem üblich, die Fasern im Anschluss an die Farb- beziehungsweise Formverändernde Behandlung einer speziellen Nachbehandlung zu unterziehen. Dabei werden, üblicherweise in Form einer Spülung, die Haare mit speziellen Wirkstoffen, beispielsweise quaternären Ammoniumsalzen oder speziellen Polymeren, behandelt. Durch diese Behandlung werden je nach Formulierung Kämmbarkeit, Halt und Fülle der Haare verbessert und die Splissrate verringert.

In jüngster Zeit wurden ferner so genannte Kombinationspräparate entwickelt, um den Aufwand der üblichen mehrstufigen Verfahren, insbesondere bei der direkten Anwendung durch Verbraucher, zu verringern. Diese Präparate enthalten neben den üblichen Komponenten, beispielsweise zur Färbung der Haare, zusätzlich Wirkstoffe, die früher den Haarnachbehandlungsmitteln vorbehalten waren. Der Konsument spart somit einen Anwendungsschritt; gleichzeitig wird der Verpackungsaufwand verringert, da ein Produkt weniger gebraucht wird. Dennoch lassen auch diese Mittel, insbesondere auf schwer zu pflegenden Fasern noch einige Wünsche hinsichtlich der pflegenden Eigenschaften offen. Die im Rahmen derartiger Kombinationspräparate einsetzbaren Wirkstoffe müssen insbesondere hinsichtlich ihrer Stabilität hohen Ansprüchen genügen, da die Färbecremes beziehungsweise Wellmittel üblicherweise einen hohen pH-Wert und die Oxidationsmittelzubereitungen einen niedrigen pH-Wert aufweisen. Ferner sind Unverträglichkeiten der verschiedenen Wirkstoffe untereinander und somit eine geringe Lagerstabilität zu vermeiden.

Insbesondere oxidative Haarfärbemittel sind trotz ihrer vorteilhaften Färbeeigenschaften für den Anwender mit Nachteilen behaftet. Einerseits führt der Einsatz der Oxidationsmittel zur Ausfärbung respektive Entwicklung der eigentlichen Färbung zu Schädigungen in der Haarstruktur und auf der Haaroberfläche. Andererseits benötigen oxidative Färbemittel in der Regel einen basischen pH-Wert zur Ausfärbung, insbesondere zwischen pH 9,0 und pH 10,5. Dies führt dazu, dass das Haar brüchig wird, seine Elastizität nachlässt und die Kämmbarkeit abnimmt. Diese Schädigung nimmt mit der Anwendungsdauer zu. Handelsübliche oxidative Färbemittel müssen meist über einen Zeitraum von 30 Minuten und länger auf die Haarfaser einwirken. Die Erhöhung der Einwirkzeit führt zu einer gesteigerten Beeinträchtigung der Haarstruktur. Weiterhin wird der basische pH-Wert häufig mit Ammoniak als Alkalisierungsmittel eingestellt, da ammoniakhaltige Färbemittel zusätzliche Vorteile hinsichtlich der Färbeleistung besitzen. Für den Anwender besitzt ein solches Färbemittel neben einen intensiven, unangenehmen Geruch jedoch den Nachteil, dass es durch Ammoniak zu Reizungen von Augen, Kopfhaut oder Schleimhäuten kommen kann, wodurch Sensibilisierungen oder gar allergische Reaktionen hervorgerufen werden können.

FR 2820032A1 offenbart Oxidationsfärbemittel, die ein Terpolymer enthalten, das unter anderem Monomere von Dimethylaminoethyl-methacrylamid sowie mindestens ein Monomer mit einer $C_9$-$C_{30}$-Alkylkette enthält. Optional können diese Färbemittel mindestens ein weiteres amphoteres oder kationisches Copolymer enthalten, darunter auch ein Copolymer gemäß Formel (I) der vorliegenden Erfindung.

Obwohl Färbungen mit oxidativen Haarfärbemitteln gegenüber Färbungen mit anderen Färbemitteln, insbesondere direktziehenden Farbstoffen, deutliche Vorteile bezüglich ihrem Auswaschverhalten und damit bezüglich des Farberhalts besitzen, besteht auch weiterhin ein Verbesserungsbedarf im Bezug auf Farberhalt und Stabilität der Färbungen auf dem Haar. Sollen ergraute oder weiße Haare gefärbt werden, besteht außerdem weiterhin Bedarf nach Färbemittel, die eine effiziente Grauabdeckung erwirken. Gleichzeitig sollen die Mittel unterschiedlich vorbehandeltes Haar gleichmäßig färben und daher über ein sehr gutes Egalisierungsvermögen verfügen.

In nicht vorhersehbarer Weise wurde nun gefunden, dass farbverändernde Mittel, die ein oxidatives Farbstoffvorprodukt enthalten, hinsichtlich ihres Egalisiervermögens und ihres Vermögen zur Grauabdeckung signifikant verbessert werden können, wenn sie zusätzlich ein kationisierbares Copolymer mit einer basischen Aminoalkyl-Seitenkette enthalten.

Ein erster Gegenstand der Erfindung ist daher ein Mittel zum Färben keratinischer Fasern, insbesondere menschlicher Haare, enthaltend in einem kosmetischen Träger mindestens ein Oxidationsfarbstoffvorprodukt, dadurch gekennzeichnet, dass das Färbemittel zusätzlich mindestens ein nicht permanent ionisches Copolymer enthält, welches sich ableitet von mindestens einem kationisierbaren Monomer der allgemeinen Formel (I),

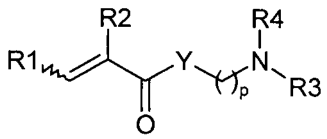

(I),

worin R1 und R2 jeweils unabhängig voneinander für Wasserstoff oder eine Methylgruppe stehen, R3 und R4 jeweils unabhängig voneinander für Alkylgruppen mit 1 bis 4 Kohlenstoff-Atomen stehen, Y für eine NH-Gruppe und p für eine ganze Zahl von 2 bis 5 steht und weiterhin mindestens ein amphoteres Tensid enthalten ist.

Unter keratinischen Fasern oder auch Keratinfasern sind dabei Pelze, Wolle, Federn und insbesondere menschliche Haare zu verstehen. Obwohl die erfindungsgemäßen Mittel in erster Linie zum Färben von Keratinfasern geeignet sind, steht prinzipiell einer Verwendung auch auf anderen Gebieten nichts entgegen.

Die erfindungsgemäßen Mittel enthalten die Wirkstoffe in einem kosmetischen Träger. Dieser kosmetische Träger ist im Sinne der Erfindung ist wässrig, alkoholisch oder wässrigalkoholisch. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Unter wässrig-alkoholischen Trägern sind im Sinne der vorliegenden Erfindung wasserhaltige Zusammensetzungen, enthaltend 3 bis 70 Gew.-% eines $C_1$-$C_4$-Alkohols, bezogen auf das Gesamtgewicht der Anwendungsmischung, insbesondere Ethanol bzw. Isopropanol, zu verstehen. Die erfindungsgemäßen Mittel können zusätzlich weitere organische Lösungsmittel, wie beispielsweise 4-Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel. Ein wässriger Träger enthält im Sinne der Erfindung mindestens 30 Gew.-%, insbesondere mindestens 50 Gew.-% Wasser, bezogen auf das Gesamtgewicht der Anwendungsmischung.

Als ersten wesentlichen Inhaltsstoff enthält das erfindungsgemäße Mittel mindestens ein Oxidationsfarbstoffvorprodukt.

In einer bevorzugten Ausführungsform enthalten die erfindungsgemäßen Mittel mindestens ein Oxidationsfarbstoffvorprodukt vom Entwicklertyp und/oder Kupplertyp enthalten. Vorzugsweise enthalten die erfindungsgemäßen Färbemittel mindestens ein Oxidationsfarbstoffvorprodukt vom Entwicklertyp und mindestens ein Oxidationsfarbstoffvorprodukt vom Kupplertyp.

Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Hydrobromide oder der Sulfate einzusetzen.

Nachfolgend werden erfindungsgemäße Entwicklerkomponenten eingehender vorgestellt.

Besonders bevorzugt sind p-Phenylendiaminderivate. Besonders bevorzugte p-Phenylendiamine werden ausgewählt aus einer oder mehrerer Verbindungen der Gruppe, die gebildet wird, aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Di-propyl-p-phenylendiamin, 4-Amino-3-methyl-(N,N-diethyl)-anilin, N,N-Bis-(2-hydroxyethyl)-p-phenylendiamin, 4-N,N-Bis-(2-hydroxyethyl)-amino-2-methylanilin, 4-N,N-Bis-(2-hydroxy-ethyl)amino-2-chloranilin, 2-(2-Hydroxyethyl)-p-phenylendiamin, 2-(1,2-Dihydroxyethyl)-p-phenylendiamin, 2-Fluor-p-phenylendiamin, 2-Isopropyl-p-phenylendiamin, N-(2-Hydroxy-propyl)-p-phenylendiamin, 2-Hydroxymethyl-p-phenylendiamin, N,N-Dimethyl-3-methyl-p-phenylendiamin, N-Ethyl-N-2-hydroxyethyl-p-phenylendiamin, N-(2,3-Dihydroxypropyl)-p-phenylendiamin, N-(4'-Aminophenyl)-p-phenylendiamin, N-Phenyl-p-phenylendiamin, 2-(2-Hydroxyethyloxy)-p-phenylendiamin, 2-Methoxymethyl-p-phenylendiamin, 2-(2-Acetylamino-ethyloxy)-p-phenylendiamin, N-(2-Methoxyethyl)-p-phenylendiamin, N-(4-Amino-3-methyl-phenyl)-N-[3-(1H-imidazol-1-yl)propyl]amin, 5,8-Diaminobenzo-1,4-dioxan sowie ihren physiologisch verträglichen Salzen. Erfindungsgemäß ganz besonders bevorzugte p-Phenylendiaminderivate sind ausgewählt aus mindestens einer Verbindung der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1H-imidazol-1-yl)propyl]amin, 2-Methoxymethyl-p-phenylendiamin sowie den physiologisch verträglichen Salzen dieser Verbindungen.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind. Bevorzugte zweikernige Entwicklerkomponenten werden insbesondere aus mindestens einer der folgenden Verbindungen ausgewählt: N,N'-Bis-(2-hydroxyethyl)-N,N'-bis-(4'-aminophenyl)-1,3-diaminopropan-2-ol, N,N'-Bis-(2-hydroxyethyl)-N,N'-bis-(4'-aminophenyl)ethylendiamin, N,N'-Bis-(4'-aminophenyl)tetramethylendiamin, N,N'-Bis-(2-hydroxyethyl)-N,N'-bis-(4'-aminophenyl)-tetramethylendiamin, N,N'-Bis-(4-(methyl-amino)phenyl)tetramethylendiamin, N,N'-Diethyl-N,N'-bis-(4'-amino-3'-methylphenyl)ethylendiamin, Bis-(2-hydroxy-5-aminophenyl)methan, N,N'-Bis-(4'-aminophenyl)-1,4-diazacyclo-heptan, N,N'-Bis-(2-hydroxy-5-aminobenzyl)piperazin, N-(4'-Aminophenyl)-p-phenylendiamin und 1,10-Bis-(2',5'-diaminophenyl)-1,4,7,10-tetraoxadecan sowie ihre physiologisch verträglichen Salze. Ganz besonders bevorzugte zweikernige Entwicklerkomponenten werden ausgewählt unter N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan oder eines der physiologisch verträglichen Salze dieser Verbindungen.

Weiterhin kann es erfindungsgemäß bevorzugt sein, als Entwicklerkomponente ein p-Aminophenolderivat oder eines seiner physiologisch verträglichen Salze einzusetzen. Bevorzugte p-Aminophenole sind insbesondere p-Aminophenol, N-Methyl-p-aminophenol, 4-Amino-3-methylphenol, 4-Amino-3-fluorphenol, 2-Hydroxymethylamino-4-aminophenol, 4-Amino-3-hydroxymethylphenol, 4-Amino-2-(2-hydroxyethoxy)-phenol, 4-Amino-2-methylphenol, 4-Amino-2-hydroxymethylphenol, 4-Amino-2-methoxymethyl-phenol, 4-Amino-2-aminomethyl-phenol, 4-Amino-2-(2-hydroxyethyl-aminomethyl)-phenol, 4-Amino-2-(1,2-dihydroxyethyl)-phenol, 4-Amino-2-fluorphenol, 4-Amino-2-chlorphenol, 4-Amino-2,6-dichlorphenol, 4-Amino-2-(diethylaminomethyl)phenol sowie ihre physiologisch verträglichen Salze. Ganz besonders bevorzugte Verbindungen sind p-Aminophenol, 4-Amino-3-methylphenol, 4-Amino-2-amino-methylphenol, 4-Amino-2-(1,2-dihydroxyethyl)-phenol und 4-Amino-2-(diethylaminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise aus Pyrimidinderivaten, Pyrazolderivaten, Pyrazolopyrimidin-Derivaten bzw. ihren physiologisch verträglichen Salzen. Bevorzugte Pyrimidin-derivate sind insbesondere die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin. Bevorzugte Pyrazol-derivate sind insbesondere die Verbindungen, die ausgewählt werden unter 4,5-Di-amino-1-methylpyrazol, 4,5-Diamino-1-(2-hydroxyethyl)pyrazol, 3,4-Diaminopyrazol, 4,5-Di-amino-1-(4'-chlorbenzyl)pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert-butyl-1-methylpyrazol, 4,5-Diamino-1-tert.-butyl-3-methylpyrazol, 4,5-Diamino-1-(2-hydroxyethyl)-3-methylpyrazol, 4,5-Diamino-1-ethyl-3-methylpyrazol, 4,5-Diamino-1-ethyl-3-(4'-methoxyphenyl)pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2-aminoethyl)amino-1,3-dimethylpyrazol, sowie deren physiologisch verträglichen Salze. Bevorzugte Pyrazolopyrimidin-Derivate sind insbesondere Pyrazolo[1,5-a]pyrimidin-3,7-diamin, 2,5-Dimethyl-pyrazolo[1,5-a]pyrimidin-3,7-diamin, Pyrazolo[1,5-a]pyrimidin-3,5-diamin, 2,7-Dimethyl-pyrazolo[1,5-a]pyrimidin-3,5-diamin, 3-Aminopyrazolo[1,5-a]pyrimidin-7-ol, 3-Aminopyrazolo[1,5-a]pyrimidin-5-ol, 2-(3-Aminopyrazolo[1,5-a]pyrimidin-7-ylamino)ethanol, 2-(7-Aminopyrazolo[1,5-a]pyrimidin-3-ylamino)ethanol, 2-[(3-Aminopyrazolo[1,5-a]pyrimidin-7-yl)-(2-hydroxyethyl)-amino]-ethanol, 2-[(7-Aminopyrazolo[1,5-a]pyrimidin-3-yl)-(2-hydroxyethyl)amino]ethanol, 5,6-Dimethylpyrazolo[1,5-a]pyrimidin-3,7-diamin, 2,6-Dimethylpyrazolo-[1,5-a]pyrimidin-3,7-diamin, 3-Amino-7-dimethylamino-2,5-dimethylpyrazolo[1,5-a]pyrimidin sowie ihre physiologisch verträglichen Salze und ihre tautomeren Formen.

Ganz besonders bevorzugte Entwicklerkomponenten werden ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methyl-phenyl)-N-[3-(1 H-imidazol-1-yl)propyl]amin, N,N'-Bis-(2-hydroxyethyl)-N,N'-bis-(4-amino-phenyl)-1,3-diamino-propan-2-ol, Bis-(2-hydroxy-5-aminophenyl)methan, 1,3-Bis-(2,5-di-aminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol und 4-Amino-2-(diethylamino-methyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, sowie den physiologisch verträglichen Salzen dieser Verbindungen. Die Entwicklerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Kupplerkomponente zusätzlich mindestens eine Entwicklerkomponente zum Einsatz kommt.

Erfindungsgemäß verwendbaren m-Aminophenole bzw. deren Derivate werden bevorzugt ausgewählt aus mindestens einer Verbindung aus der Gruppe, die gebildet wird aus 3-Amino-phenol, 5-Amino-2-methylphenol, N-Cyclopentyl-3-aminophenol, 3-Amino-2-chlor-6-methyl-phenol, 2-Hydroxy-4-aminophenoxyethanol, 2,6-Dimethyl-3-aminophenol, 3-Trifluoroacetyl-amino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-Diethylaminophenol, N-Cyclopentyl-3-aminophenol, 1,3-Dihydroxy-5-(methylamino)benzol, 3-Ethylamino-4-methylphenol, 2,4-Dichlor-3-aminophenol und den physiologisch verträglichen Salzen aller vorstehend genannten Verbindungen.

Erfindungsgemäß verwendbaren m-Diaminobenzole bzw. deren Derivate werden bevorzugt ausgewählt aus mindestens einer Verbindung aus der Gruppe, die gebildet wird aus m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl} amino)ethanol, 2-({3-[(2-Hydroxyethyl)amino]-2-methoxy-5-methylphenyl}-amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)aminobenzol und den physiologisch verträglichen Salzen aller vorstehend genannten Verbindungen.

Erfindungsgemäß verwendbaren o-Diaminobenzole bzw. deren Derivate werden bevorzugt ausgewählt aus mindestens einer Verbindung aus der Gruppe, die gebildet wird aus 3,4-Diaminobenzoesäure und 2,3-Diamino-1-methylbenzol und den physiologisch verträglichen Salzen aller vorstehend genannten Verbindungen.

Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol.

Erfindungsgemäß verwendbaren Pyridinderivate werden bevorzugt ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Di-amino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin, und den physiologisch verträglichen Salzen der vorgenannten Verbindungen.

Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxy-naphthalin, 2,7-Dihydroxynaphthalin und 2,3-Dihydroxynaphthalin.

Erfindungsgemäß verwendbaren Indolderivate werden ausgewählt aus mindestens einer Verbindung der Gruppe, die gebildet wird aus 4-Hydroxyindol, 6-Hydroxyindol und 7-Hydroxy-indol und den physiologisch verträglichen Salzen

der vorgenannten Verbindungen.

Erfindungsgemäß verwendbaren Indolinderivate werden bevorzugt ausgewählt aus mindestens einer Verbindung der Gruppe, die gebildet wird aus 4-Hydroxyindolin, 6-Hydroxyindolin und 7-Hydroxyindolin und den physiologisch verträglichen Salzen der vorgenannten Verbindungen.

Bevorzugte Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Di-amino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und den physiologisch verträglichen Salzen der vorgenannten Verbindungen.

Erfindungsgemäß besonders bevorzugte Kupplerkomponenten werden ausgewählt unter 3-Aminophenol, 5-Amino-2-methylphenol, 3-Amino-2-chlor-6-methylphenol, 2-Hydroxy-4-amino-phenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)-ethanol, 1,3-Bis(2,4-diaminophenoxy)propan, 1-Methoxy-2-amino-4-(2-hydroxyethylamino)-benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxy-ethyl)amino]-2-methoxy-5-methylphenyl}amino)ethanol, 2-({3-[(2-Hydroxyethyl)amino]-4,5-di-methylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen.

Die Kupplerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet. Die Oxidationsfarbstoffvorprodukte werden im Rahmen der vorliegenden Erfindung vorzugsweise in einer Menge von 0,005 bis 20 Gew.-%, bevorzugt von 0,05 bis 5 Gew.-% und besonders bevorzugt von 0,1 bis 5 Gew.-%, jeweils bezogen auf das anwendungsbereite Oxidationsfärbemittel, verwendet. Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 zu 0,5 bis 1 zu 3, insbesondere 1 zu 1 bis 1 zu 2, stehen können.

Als weiteren zwingenden Inhaltsstoff enthält das Mittel ein nicht permanent ionisches Copolymer, welches sich ableitet von mindestens einem kationisierbaren Monomer der allgemeinen Formel (I)

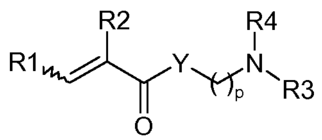

(I),

worin R1 und R2 jeweils unabhängig voneinander für Wasserstoff oder eine Methylgruppe stehen, R3 und R4 jeweils unabhängig voneinander für Alkylgruppen mit 1 bis 4 Kohlenstoff-Atomen stehen, Y für eine NH-Gruppe und p für eine ganze Zahl von 2 bis 5 steht.

Der Begriff "permanent ionisch" ist dabei so zu verstehen, dass das Copolymer eine permanente Ladung trägt, wie sie beispielsweise durch Alkylierung eines basischen, tertiären Stickstoffs erfolgen kann. Copolymere, die sich von Monomeren gemäß Formel (I) und mindestens einem weiteren Monomeren, welches eine permanente Ladung trägt, ableiten, sind daher im Rahmen der vorliegenden Anmeldung nicht erfindungsgemäß. Insbesondere sind daher permanent kationische Polymere, wie zum Beispiel Polyquaternium-55, nicht von Umfang der vorliegenden Anmeldung umfasst.

"Nicht permanent ionische" Copolymere liegen im Gegensatz dazu nicht über den gesamten, physiologisch verträglichen pH-Bereich geladen vor, sondern stehen in einem vom pH-Wert abhängigen Gleichgewicht mit ihrer ungeladenen, nicht ionischen Form.

Der Begriff "kationisierbar" ist im Sinne der Anmeldung zu verstehen, dass die Verbindung einen basischen Stickstoff enthält, welcher unter physiologisch verträglichen pH-Bedingungen befähigt ist, protoniert zu werden und damit in eine kationische Form überzugehen. Je nach pH-Wert wird dabei die protonierte, kationische oder die basische, ungeladene Form überwiegen.

Erfindungsgemäß bevorzugte Copolymere sind dadurch gekennzeichnet, dass das Copolymer sich mindestens von einem kationisierbaren Monomer der allgemeinen Formel (I), worin R2 für eine Methylgruppe und p für die Zahl 2 oder 3 steht. Beispiele für Monomere dieser Art sind Methacrylsäure-2-aminoethylamid, Methacrylsäure-3-aminopropylamid, Methacrylsäure-2-(methylamino)ethylamid, Methacrylsäure-3-(methylamino)propylamid, Methacrylsäure-2-(ethylamino)ethylamid, Methacrylsäure-3-(ethylamino)propylamid, Methacrylsäure-2-(dimethylamino)ethylamid, Methacrylsäure-3-(dimethylamino)propylamid, Methacrylsäure-2-(diethylamino)ethylamid, Methacrylsäure-3-(diethylamino)propylamid, Methacrylsäure-2-(ethylmethylamino)ethylamid und Methacrylsäure-3-(ethylmethylamino)propylamid.

Besonders bevorzugte Copolymere enthalten erfindungsgemäß ein Monomer gemäß Formel (I), in dem R1 für Wasserstoff, R2 für Methyl, und mindestens einer der Reste R3 oder R4 für eine Methylgruppe steht. Es ist erfindungsgemäß weiterhin besonders vorteilhaft, wenn p für die Zahl 2 oder 3, insbesondere jedoch 3 steht. Beispiele für solche Monomere dieser Art sind Methacrylsäure-2-(methylamino)-ethylamid, Methacrylsäure-3-(methylamino)propylamid, Methacrylsäure-2-(dimethylamino)-ethylamid, Methacrylsäure-3-(dimethylamino)propylamid, Methacrylsäure-2-(ethylmethyl-amino)ethylamid und Methacrylsäure-3-(ethylmethylamino)propylamid.

Eine ganz besonders bevorzugte Ausführungsform liegt dann vor, wenn das Mittel der vorliegenden Anmeldung ein Copolymer enthält, das sich mindestens von einem kationisierbaren Monomer der allgemeinen Formel (I) ableitet, worin R1 für Wasserstoff, R2, R3 und R4 jeweils für eine Methylgruppe, Y für eine NH-Gruppe und p für die Zahl 3 steht :Methacrylsäure-3-(dimethylaminopropyl)amid.

Das erfindungsgemäße kationisierbare Copolymer enthält weiterhin mindestens ein ungeladenes Monomer mit einer polymerisierbaren Ethenylgruppe, bevorzugt zusätzlich mit einer Amidfunktion. Beispiele für solche Monomere sind Acrylsäureamid, Methacrylsäureamid, Crotonsäureamid, Acrylsäure-N-methylamid, Methacrylsäure-N-methylamid, Crotonsäure-N-methylamid, Acrylsäure-N,N-dimethylamid, Methacrylsäure-N,N-dimethylamid, Crotonsäure-N,N-dimethylamid, Acrylsäure-N-ethylamid, Methacrylsäure-N-ethylamid, Crotonsäure-N-ethylamid, Ameisensäure-N-vinylamid, Essigsäure-N-vinylamid, Ameisensäure-N-methyl-N-vinylamid, Essigsäure-N-methyl-N-vinylamid, N-Vinyl-pyrrolidin-2-on, N-Vinyl-piperidin-2-on und N-Vinyl-azepan-2-on.

Erfindungsgemäß besonders bevorzugte Copolymer zeichnen sich dadurch aus, dass sie sich als weiterem Monomer von mindestens einem N-Vinyl-Lactam ableiten. Ganz besonders bevorzugte Copolymer leiten von mindestens einem N-Vinyl-Lactam, ausgewählt aus N-Vinyl-pyrrolidin-2-on und N-Vinyl-azepan-2-on, als weiteres Monomer ab.

Beispiele für kommerziell erhältliche Copolymere gemäß vorliegender Erfindung werden unter dem Handelsnamen Copolymer 937 (Vinylpyrrolidinon/Dimethylaminomethacrylat Copolymer; ISP) und Styleze® CC10 (Vinylpyrrolidinon/ Dimethylaminopropylmethacrylamid Copolymer; ISP) vertrieben.

Kommerziell erhältliche Terpolymere, die ebenfalls erfindungsgemäß einsetzbar sind, werden unter dem Handelsnamen Advantage® (Vinylcaprolactam/ Vinylpyrrolidinon/ Dimethylaminoethylmethacrylat Terpolymer; ISP) oder Aquaflex® SF 40 (Vinylpyrrolidinon/ Vinylcaprolactam/ Dimethylaminopropylmethacrylamid Terpolymer; ISP) vertrieben.

Ganz besonders vorteilhafte Mittel sind dadurch gekennzeichnet, dass das Copolymer sich mindestens von einem kationisierbaren Monomer der allgemeinen Formel (I), worin R1 für Wasserstoff, R2, R3 und R4 jeweils für eine Methylgruppe, Y für eine NH-Gruppe und p für die Zahl 3 steht, und mindestens N-Vinyl-pyrrolidin-2-on ableitet.

Kommerziell ist ein solches Copolymer unter dem Namen Styleze® CC10 (Vinylpyrrolidinon/ Dimethylaminopropylmethacrylamid Copolymer; ISP) erhältlich.

Erfindungsgemäß vorteilhaft als Copolymere sind solche, die ein Molekulargewicht von 100,000 bis 500,000 g/mol, insbesondere von 250,000 bis 350,000 g/mol besitzen.

Das erfindungsgemäße Mittel enthält weiterhin zwingend mindestens ein amphoteres Tensid.

Unter amphoteren Tensiden werden solche oberflächenaktiven Verbindungen verstanden, die außer einer $C_8$-$C_{24}$-Alkyl- oder -Acylgruppe im Molekül mindestens eine freie Aminogruppe und mindestens eine Carbonsäure- oder Sulfonsäure-Gruppe enthalten und zur Ausbildung innerer Salze befähigt sind. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe.

Erfindungsgemäß bevorzugte Mittel sind dadurch gekennzeichnet, dass als amphoteres Tensid ein Tensid gemäß Formel (II) und/oder dessen physiologisch verträgliches Salz,

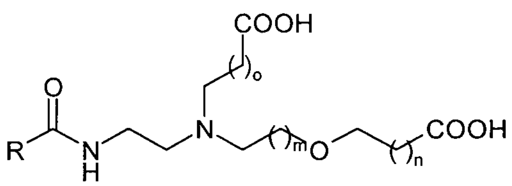

(II),

worin R für eine lineare oder verzweigte, gesättigte oder ungesättigte $C_{10}$-$C_{22}$-Alkylgruppe steht und m, n und o jeweils unabhängig voneinander für eine ganze Zahl 0, 1, 2 oder 3 steht, enthalten ist.

Besonders bevorzugte amphotere Tenside werden unter der INCI-Bezeichnung Disodium Cocoamphodipropionate mit den Handelsnamen Miranol® C2M SF conc. (Rhodia), Amphoterge® K-2 (Lonza) und Monateric® CEM-38 (Unichema) und Bezeichnung Disodium Cocoamphodiacetate mit den Handelsnamen Dehyton® (Cognis), Miranol® C2M (Rhodia) und Ampholak® XCO 30 (Akzo Nobel) vermarktet. Ganz besonders bevorzugte ist Disodium Cocoamphodipropionate.

Der Zusatz im erfindungsgemäßen Mittel bewirkt eine hervorragende Avivage, insbesondere eine verbesserte Nasskämmbarkeit sowie eine Verbesserung des Farberhalts.

Die amphoteren Tenside sind in dem erfindungsgemäßen Mittel bevorzugt in einer Menge von 0,05 bis 30 Gew.-%, besonders bevorzugt von 0,1 bis 20 Gew.-%, jeweils bezogen auf das Gewicht des Mittels, enthalten.

In einer bevorzugten Ausführungsform enthält das erfindungsgemäße Mittel zur Farbveränderung keratinischer Fasern eine zusätzliche farbgebende Komponente. Die zusätzliche farbgebende Komponente wird bevorzugt ausgewählt aus mindestens einem direktziehenden Farbstoff und/oder aus Oxofarbstoffvorprodukten und/oder aus mindestens einer Vorstufe naturanaloger Farbstoffe.

Erfindungsgemäß besonders vorteilhafte Mittel sind dadurch gekennzeichnet, dass sie zusätzlich mindestens ein direktziehender Farbstoff enthalten. Bei direktziehenden Farbstoffen handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole. Die direktziehenden Farbstoffe werden jeweils bevorzugt in einer Menge von 0,001 bis 20 Gew.-%, bezogen auf die gesamte Anwendungszubereitung, eingesetzt. Die Gesamtmenge an direktziehenden Farbstoffen beträgt vorzugsweise höchstens 20 Gew.-%. Direktziehende Farbstoffe können in anionische, kationische und nichtionische direktziehende Farbstoffe unterteilt werden.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 bekannten Verbindungen.

Bevorzugte kationische direktziehende Farbstoffe sind dabei kationische Triphenylmethanfarbstoffe, wie beispielsweise Basic Blue 7, Basic Blue 26, Basic Violet 2 und Basic Violet 14, aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie direktziehende Farbstoffe, die einen Heterocyclus enthalten, der mindestens ein quaternäres Stickstoffatom aufweist. Die Verbindungen, die auch unter den Bezeichnungen Basic Yellow 87, Basic Orange 31 und Basic Red 51 bekannt sind, sind ganz besonders bevorzugte kationische direktziehende Farbstoffe.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureido-ethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu und Alkannawurzel enthalten sind.

Als erfindungsgemäße farbverändernde Komponente können in einer weiteren Ausführungsform auch Oxofarbstoffvorprodukte eingesetzt werden. Oxofarbstoffvorprodukte werden bevorzugt als Kombination aus mindestens einer Verbindung, die mindestens eine reaktive Carbonyl-Gruppe enthält (Komponente (Oxo1)), mit mindestens einer Verbindung (Komponente Oxo2), ausgewählt aus CH-aciden Verbindungen und/oder ausVerbindungen mit primärer oder sekundärer Aminogruppe oder Hydroxygruppe, ausgewählt aus mindestens einer Verbindung der Gruppe, die gebildet wird aus primären oder sekundären aromatischen Aminen, stickstoffhaltigen heterocyclischen Verbindungen und aromatischen Hydroxyverbindungen, eingesetzt.

In einer weiteren Ausführungsform der vorliegenden Erfindung wird die farbverändernde Komponente aus Farbstoffvorstufen naturanaloger Farbstoffe ausgewählt. Als Farbstoffvorstufen naturanaloger Farbstoffe werden bevorzugt solche Indole und Indoline eingesetzt, die mindestens zwei Gruppen ausgewählt aus Hydroxy- und/oder oder Aminogruppen, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer weiteren Ausführungsform enthalten die Färbemittel mindestens ein Indol- und/oder Indolinderivat. Erfindungsgemäße Zusammensetzungen, die Vorstufen naturanaloger Farbstoffe enthalten, werden bevorzugt als luftoxidative Färbemittel verwendet. In dieser Ausführungsform werden die besagten Zusammensetzungen folglich nicht mit einem

zusätzlichen Oxidationsmittel versetzt. Die Farbstoffvorstufen naturanaloger Farbstoffe werden jeweils bevorzugt in einer Menge von 0,001 bis 5 Gew.-%, bezogen auf die gesamte Anwendungszubereitung, eingesetzt. Die Gesamtmenge an direktziehenden Farbstoffen beträgt vorzugsweise höchstens 3 Gew.-%. Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin sowie 5,6-Dihydroxyindolin-2-carbonsäure, insbesondere das 5,6-Dihydroxyindolin. Besonders bevorzugte Derivate des Indols sind 5,6-Dihydroxyindol, N-Methyl-5,6-dihydroxyindol, N-Ethyl-5,6-dihy-droxyindol, N-Propyl-5,6-dihydroxyindol, N-Butyl-5,6-dihydroxyindol, 5,6-Dihydroxyindol-2-carbonsäure, insbesondere das 5,6-Dihydroxyindol.

Es ist nicht erforderlich, dass Oxidationsfarbstoffvorprodukte, Oxofarbstoffvorprodukte, direktziehende Farbstoffe oder naturanaloge Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Weiterhin hat es sich als besonders vorteilhaft herausgestellt, wenn das erfindungsgemäße Mittel in Form einer fließfähigen Zubereitung vorliegt. Bevorzugt enthalten die fließfähigen Zubereitungen zusätzlich als oberflächenaktive Substanz einen Emulgator bzw. ein weiteres Tensid, wobei oberflächenaktive Substanzen je nach Anwendungsgebiet als Tenside oder als Emulgatoren bezeichnet werden und aus anionischen, kationischen, zwitterionischen und nichtionischen Tensiden und Emulgatoren ausgewählt sind.

Bevorzugte anionische Tenside sind Alkylsulfate, Alkylethersulfate und Ethercarbonsäuren mit 10 bis 18 C-Atomen in der Alkylgruppe und bis zu 12 Glykolethergruppen im Molekül. Erfindungsgemäß besonders bevorzugte Mittel sind jedoch dadurch gekennzeichnet, dass anionische Tenside in einem Gesamtgehalt von weniger als 1,0 Gew.-%, bevorzugt weniger als 0,5 Gew.-%, jeweils bezogen auf das Gesamtgewicht der Anwendungszubereitung, enthalten sind.

Besonders geeignete zwitterionische Tenside sind die sogenannten Betaine wie die N-Alkyl-N,N-dimethylammonium-glycinate, N-Acyl-aminopropyl-N,N-dimethylammoniumglycinate, und 2-Alkyl-3-carboxymethyl-3-hydroxyethyl-imidazoline sowie das Kokosacylaminoethylhydroxy-ethylcarboxymethylglycinat. Ein bevorzugtes zwitterionisches Tensid ist das unter der INCI-Bezeichnung Cocamidopropyl Betaine bekannte Fettsäureamid-Derivat.

Weiterhin hat es sich als vorteilhaft erwiesen, wenn die erfindungsgemäßen Aufhellmittel nichtionogene grenzflächenaktive Stoffe enthalten. Als nichtionische Tenside eignen sich insbesondere $C_8$-$C_{22}$-Alkylmono- und -oligoglycoside und deren ethoxylierte Analoga. Als weitere bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Die nichtionischen oder zwitterionischen Tenside werden in Mengen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß bevorzugt sind ebenfalls kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride. Weitere erfindungsgemäß verwendbare kationische Tenside stellen die quaternisierten Proteinhydrolysate dar. Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyldimethylamin dar. Ebenfalls sehr gut biologisch abbaubar sind quaternäre Esterverbindungen, sogenannte "Esterquats". Bei Esterquats handelt es sich um bekannte Stoffe, die sowohl mindestens eine Esterfunktion als auch mindestens eine quartäre Ammoniumgruppe als Strukturelement enthalten. Solche Produkte werden beispielsweise unter den Warenzeichen Stepantex®, Dehyquart® und Armocare® vertrieben. Die Produkte Armocare® VGH-70, ein N,N-Bis(2-Palmitoyloxyethyl)dimethylammoniumchlorid, sowie Dehyquart® F-75, Dehyquart® C-4046, Dehyquart® L80 und Dehyquart® AU-35 sind ebenfalls Beispiele für solche Esterquats. Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

In einer bevorzugten Ausführungsform können Mittel, enthaltend nicht-ionische, zwitterionische und/oder kationische Tenside sowie deren Mischungen, bevorzugt sein.

In einer weiteren bevorzugten Ausführungsform kann die Wirkung des erfindungsgemäßen Wirkstoffes durch Emulgatoren gesteigert werden. Solche Emulgatoren sind beispielsweise Anlagerungsprodukte von 4 bis 30 Mol Ethylenoxid und/oder 0 bis 5 Mol Propylenoxid an lineare Fettalkohole, an Fettsäuren und an Alkylphenole; $C_{12}$-$C_{22}$-Fettsäuremono- und -diester von Anlagerungsprodukten von 1 bis 30 Mol Ethylenoxid an Polyole, insbesondere an Glycerin; Ethylenoxid- und Polyglycerin-Anlagerungsprodukte an Methylglucosid-Fettsäureester, Fettsäurealkanolamide und Fettsäureglucamide; $C_8$-$C_{22}$-Alkylmono- und -oligoglycoside und deren ethoxylierte Analoga; Gemische aus Alkyl-(oligo)-glucosiden und Fettalkoholen; Anlagerungsprodukte von 5 bis 60 Mol Ethylenoxid an Rizinusöl und gehärtetes Rizinusöl; Partialester von Polyolen mit gesättigten Fettsäuren; Sterine aus tierischem Gewebe (Zoosterine; Beispiele: Cholesterin, Lanosterin), aus pflanzlichen Fetten (Phytosterine; Beispiele: Ergosterin, Stigmasterin und Sitosterin) und aus Pilzen und Hefen (Mykosterine); Phospholipide, vor allem Glucose-Phospolipide wie Lecithine bzw. Phosphatidylcholine; Fettsäureester von Zuckern und Zuckeralkoholen, wie Sorbit; Polyglycerine und Polyglycerinderivate; lineare und verzweigte Fettsäuren mit 8 bis 30 C-Atomen und deren Na-, K-, Ammonium-, Ca-, Mg- und Zn-Salze. Die erfindungsgemäßen Mittel enthalten die Emulgatoren bevorzugt in Mengen von 0,1 bis 25 Gew.-%, insbesondere 0,5 bis 15 Gew.-%, bezogen auf die Gesamtmenge des anwendungsbereiten Mittel.

Im Falle der oxidativen Färbungen kann die Entwicklung der Farbe grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Dieser Aufhelleffekt kann unabhängig von der Färbemethode gewünscht sein. Als Oxidationsmittel kommen Persulfate, Peroxodisulfate, Chlorite, Hypochlorite und insbesondere Wasserstoffperoxid oder dessen Anlagerungsprodukte an Harnstoff, Melamin sowie Natriumborat in Frage.

Bevorzugt wird als Oxidationsmittel Wasserstoffperoxid eingesetzt. Bevorzugt beträgt die Menge an Wasserstoffperoxid im anwendungsbereiten Mittel 0,5 bis 12 Gew.-%, bevorzugt 1 bis 8 Gew.-%, jeweils bezogen auf das anwendungsbereite Mittel.

Solche Oxidationsmittelzubereitungen sind vorzugsweise wässrige, fließfähige Oxidationsmittelzubereitungen. Dabei sind bevorzugte Zubereitungen dadurch gekennzeichnet, dass die fließfähige Oxidationsmittelzubereitung - bezogen auf ihr Gewicht - 40 bis 90 Gew.-%, vorzugsweise 50 bis 85 Gew.-%, besonders bevorzugt 55 bis 80 Gew.-%, weiter bevorzugt 60 bis 77,5 Gew.-% und insbesondere 65 bis 75 Gew.-% Wasser enthält.

Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z. B. bestimmte Enzyme, Iodide, Chinone oder Metallionen.

Geeignete Enzyme sind z. B. Peroxidasen, die die Wirkung geringer Mengen an Wasserstoffperoxid deutlich verstärken können. Ein Einsatz bestimmter Metallionen oder -komplexe kann ebenfalls bevorzugt sein, um intensive Färbungen zu erhalten.

Das eigentliche Haarfärbemittel wird zweckmäßigerweise unmittelbar vor der Anwendung durch Mischung der Zubereitung des Oxidationsmittels mit der Zubereitung, enthaltend die Farbstoffvorprodukte sowie das erfindungsgemäße Copolymer, hergestellt. Das dabei entstehende gebrauchsfertige Haarfärbepräparat sollte bevorzugt einen pH-Wert im Bereich von 6 bis 12 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Die erfindungsgemäßen Mittel können zusätzlich auch Blondier- und/oder Bleichmittel enthalten und somit als Mittel bereitgestellt werden, die gleichzeitig färbend und aufhellend wirken. Solche Mittel werden nachfolgend als "Färbemittel", als "aufhellende Färbemittel" oder als "Färbe- und Aufhellmittel" bezeichnet.

Für die starke Aufhellung sehr dunklen Haares ist der alleinige Einsatz von Wasserstoffperoxid oder dessen Anlagerungsprodukten an organische beziehungsweise anorganische Verbindungen oftmals nicht ausreichend. In diesen Fällen wird in der Regel eine Kombination aus Wasserstoffperoxid und Persulfaten bzw. Peroxodisulfaten eingesetzt, woraus eine Steigerung des Aufhellvermögens der Mittel resultiert. Bevorzugte Peroxodisulfatsalze sind Ammoniumperoxodisulfat, Kaliumperoxodisulfat und Natriumperoxodisulfat. Die Peroxodisulfatsalze können in einer Menge von 0,1 bis 25 Gew.-%, insbesondere in einer Menge von 0,5 bis 15 Gew.-%, bezogen auf das Gesamtgewicht des anwendungsbereiten Mittels, enthalten sein.

Bei einer Anwendung von zusätzlichen Oxidationsmitteln wird das eigentliche Färbe- und/oder Aufhellmittel zweckmäßigerweise unmittelbar vor der Anwendung durch Mischung einer erfindungsgemäßen Zubereitung, enthaltend in einem kosmetischen Träger mindestens ein Oxidationsfarbstoffvorprodukt, sowie einer Zubereitung, enthaltend das zusätzliche Oxidationsmittel, insbesondere Wasserstoffperoxid, hergestellt. Wird eine starke Aufhellung gewünscht, so ist es erfindungsgemäß bevorzugt, wenn zusätzlich eine Blondierzubereitung, enthaltend mindestens ein anorganisches Persulfatsalz bzw. Peroxodisulfatsalz, der Oxidationsmittelzubereitung vor Vermischung mit der erfindungsgemäßen Färbezubereitung beigemischt wird.

Weiterhin hat es sich als vorteilhaft erweisen, wenn die Oxidationsmittelzubereitungen mindestens einen Stabilisator oder Komplexbildner enthalten. Besonders bevorzugte Stabilisatoren sind Phenacetin, Alkalibenzoate (Natriumbenzoat) und Salicylsäure.

Erfindungsgemäß bevorzugt ist auch der Einsatz von sogenannten Komplexbildnern. Komplexbilder sind Stoffe, die Metallionen komplexieren können. Bevorzugte Komplexbildner sind sogenannte Chelatkomplexbildner. Gebräuchliche und im Rahmen der vorliegenden Erfindung bevorzugte Chelatkomplexbildner sind beispielsweise Polyoxycarbonsäuren, Polyamine, Ethylendiamintetraessigsäure (EDTA), Nitrilotriessigsäure (NTA) und Hydroxyethandiphosphonsäuren bzw. deren Alkalisalze. Auch komplexbildende Polymere, also Polymere, die entweder in der Hauptkette selbst oder seitenständig zu dieser funktionelle Gruppen tragen, die als Liganden wirken können und mit geeigneten Metall-Atomen in der Regel unter Bildung von Chelat-Komplexen reagieren, sind erfindungsgemäß einsetzbar. Erfindungsgemäß bevorzugte Komplexbildner sind stickstoffhaltigen Polycarbonsäuren, insbesondere EDTA, und Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz. Bevorzugt sind die Komplexbildner im erfindungsgemäßen Mittel zu 0,05 bis 10 Gew.-%, bevorzugt zu 0,5 bis 5 Gew.-%, jeweils bezogen auf das Gesamtgewicht des anwendungsbereiten Mittels, enthalten.

Die Konfektionierung der erfindungsgemäßen Färbemittel unterliegt prinzipiell keinerlei Beschränkungen. Üblicherweise werden die erfindungsgemäßen Mittel als 1-Komponentenmittel konfektioniert, die gegebenenfalls unmittelbar vor der Anwendung mit einer zweiten Zubereitung, enthaltend beispielsweise ein Oxidationsmittel, vermischt werden. Es hat sich aber in einigen Fällen auch als bevorzugt erwiesen, wenn das Produkt als 2-Komponentenmittel konfektioniert ist. Im Rahmen einer bevorzugten Ausführungsform werden die erfindungsgemäßen Mittel daher derart konfektioniert, dass eine der erfindungswesentlichen Komponenten separat verpackt ist. Dabei spielt es erfindungsgemäß zunächst keine Rolle, welche der drei erfindungsgemäßen Komponenten separat verpackt wird; es kann aber bevorzugt sein, die Zubereitung, die das erfindungsgemäße Copolymer enthält, bis zur Anwendung separat zu verpacken.

Die Oxidationsmittelzubereitung enthält neben dem eigentlichen Oxidationsmittel weitere Hilfs- und Zusatzstoffe. So hat es sich erfindungsgemäß als bevorzugt erwiesen, wenn die Oxidationsmittelzubereitung mindestens ein Verdickungsmittel enthält. Bezüglich dieser Verdickungsmittel bestehen keine prinzipiellen Einschränkungen. Es können sowohl organische als auch rein anorganische Verdickungsmittel zum Einsatz kommen.

Die Verdickungsmittel, ausgewählt aus kationischen Polymere, Polysacchariden, nichtionischen, synthetischen Polymeren und/oder anorganischen Verdickungsmitteln, sind in den erfindungsgemäßen Mitteln bevorzugt in einer Menge von 0,01 bis 10 Gew.-%, besonders bevorzugt von 0,1 bis 5 Gew.-%, jeweils bezogen auf das Gewicht des Mittels, enthalten.

Ferner können die erfindungsgemäßen Mittel weitere Wirk-, Hilfs- und Zusatzstoffe enthalten: nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane; kationische Polymere wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidinon-Copolymere, Vinylpyrrolidinon-Imidazolinium-methochlorid-Copolymere und quaternierter Polyvinylalkohol; zwitterionische und amphotere Polymere wie beispielsweise Acrylamidopropyl-trimethylammoniumchlorid/AcrylatCopolymere und Octylacrylamid/Methyl-methacrylat/tert-Butylaminoethylmethacrylat/2-Hydroxypropylmethacrylat-Copolymere, Diallyldimethylammoniumchlorid/Acrylat-Copolymere, t-Butylaminoethylmethacrylat/N-(1,1,3,3-Tetramethylbutyl)acrylamid/Acrylat(/Methacrylat)-Copolymere; anionische Polymere wie beispielsweise Polyacrylsäuren, vernetzte Polyacrylsäuren, Vinylacetat/Crotonsäure-Copolymere, Vinylpyrrolidinon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-t-Butyl-acrylamid-Terpolymere; weitere Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol; Strukturanten wie Glucose, Maleinsäure und Milchsäure; haarkonditionierende Verbindungen wie Phospholipide, (Sojalecithin, Ei-Lecitin, Kephaline sowie Silikonöle); Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate; Parfümöle, Dimethylisosorbid und Cyclo-dextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe; Aminosäuren und Oligopeptide , insbesondere Arginin und/oder Serin; Lichtschutzmittel; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide, bevorzugt Sphingolipide oder Pseudoceramide; Vitamine, Provitamine und Vitaminvorstufen, insbesondere solche der Gruppen A, $B_3$, $B_5$, $B_6$, C, E, F und H; Pflanzenextrakte wie die Extrakte aus Grünem Tee, Eichenrinde, Brennnessel, Hamamelis, Hopfen, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Litchi, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel; Cholesterin; Fette und Wachse wie Fettalkohole, Bienenwachs, Montanwachs und Paraffine; Fettsäurealkanolamide; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/ Acrylamid-Copolymere; Perlglanzmittel; Pigmente; Treibmittel wie Propan-Butan-Gemische, $N_2O$ Dimethylether, $CO_2$ und Luft; Antioxidantien.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen. Die zusätzlichen Wirk- und Hilfsstoffe werden in den erfindungsgemäßen Mitteln bevorzugt in Mengen von jeweils 0,0001 bis 10 Gew.-%, insbesondere von 0,0005 bis 5 Gew.-%, bezogen auf das Gesamtgewicht der Anwendungsmischung, eingesetzt.

Erfindungsgemäß bevorzugte Mittel zur Farb- und Formveränderung der Haare sind dadurch gekennzeichnet, dass sie einen möglichst neutralen pH-Wert besitzen. Eine weitere bevorzugte Ausführungsform der vorliegenden Erfindung besteht darin, dass das anwendungsbereite Mittel einen pH-Wert zwischen 5,0 und 11,0, bevorzugt zwischen 6,0 und 10,0, insbesondere bevorzugt zwischen 8,0 und 9,5 besitzt. Üblicherweise wird der pH-Wert mit pH-Stellmitteln eingestellt. Zur Einstellung des pH-Werts sind dem Fachmann in der Kosmetik gängige Acidifizierungs- und Alkalisierungsmittel geläufig. Die zur Einstellung des pH-Wertes verwendbaren Alkalisierungsmittel werden erfindungsgemäß bevorzugt gewählt aus anorganischen Salzen, insbesondere der Alkali- und Erdalkalimetalle, organischen Alkalisierungsmitteln, insbesondere Aminen, basischen Aminosäuren und Alkanolaminen, und Ammoniak. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren. Bei den pH-Werten im Sinne der vorliegenden Erfindung handelt es sich um pH-Werte, die bei einer Temperatur von 22 °C gemessen wurden.

Ein weiterer Gegenstand der vorliegenden Erfindung ist ein Verfahren, bei dem ein erfindungsgemäßes Mittel gemäß obiger Vorgaben auf das Haar aufgetragen wird, für eine Einwirkzeit von 2 bis 45 Minuten, bevorzugt von 5 bis 30 Minuten auf dem Haar belassen wird, und anschließend das Haar ausgespült wird.

Eine weitere bevorzugte Ausführungsform des erfindungsgemäßen Verfahrens zur Farbveränderung keratinischer Fasern liegt vor, wenn eine Zusammensetzung in einem kosmetischen Träger, enthaltend mindestens ein Oxidationsfarbstoffvorprodukt sowie mindestens ein erfindungsgemäßes Copolymer und mindestens ein amphoteres Tensid, mit einer Oxidationsmittelzubereitung, enthaltend Wasserstoffperoxid, zu einer homogenen Zusammensetzung vermischt, diese auf das Haar aufgebracht wird, für eine Einwirkzeit von 2 bis 45 Minuten, bevorzugt von 5 bis 30 Minuten auf dem Haar belassen wird, und anschließend das Haar ausgespült wird.

Bezüglich weiterer bevorzugter Ausführungsformen der erfindungsgemäßen Verfahren gilt mutatis mutandis das zu den erfindungsgemäßen Mitteln Gesagte.

Die Anwendungstemperaturen bei der erfindungsgemäßen Farb- und/oder dauerhaften Formveränderung keratinischer Fasern können in einem Bereich zwischen 15 und 45 °C liegen. Nach einer Einwirkungszeit wird das Haarfärbemittel durch Ausspülen von dem zu färbenden Haar entfernt. Das Nachwaschen mit einem Shampoo kann entfallen, wenn ein stark tensidhaltiger Träger, z. B. ein Färbeshampoo, verwendet wurde.

Wie bereits erwähnt, können die erfindungsgemäßen Mittel auch direkt vor der Anwendung aus zwei oder mehreren getrennt verpackten Zubereitungen hergestellt werden. Dies bietet sich insbesondere zur Trennung inkompatibler Inhaltsstoffe an, um eine vorzeitige Reaktion zu vermeiden. Eine Auftrennung in Mehrkomponentensysteme bietet sich insbesondere dort an, wo Inkompatibilitäten der Inhaltsstoffe zu erwarten oder zu befürchten sind. Das anwendungsbereite Mittel wird bei solchen Systemen vom Verbraucher direkt vor der Anwendung durch Vermischen der Komponenten hergestellt. Ein Färbe- und/oder Aufhellungsmittel, bei dem die Oxidationsfarbstoffvorprodukte zunächst getrennt von der Oxidationsmittelzubereitung, enthaltend bevorzugt Wasserstoffperoxid, vorliegen, ist dabei bevorzugt.

Ein weiterer Gegenstand der vorliegenden Erfindung ist daher eine Mehrkomponentenverpackungseinheit (Kit-of-Parts), enthaltend mindestens zwei getrennt konfektionierte Container, wobei ein Container eine Färbemischung in einem kosmetischen Träger, enthaltend mindestens eine Oxidationsfarbstoffvorprodukt, mindestens ein nicht permanent ionisches Copolymer, welches sich ableitet von mindestens einem kationisierbaren Monomer der allgemeinen Formel (I),

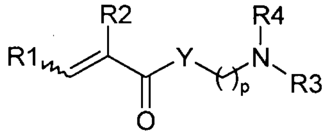

(I),

worin R1 und R2 jeweils unabhängig voneinander für Wasserstoff oder eine Methylgruppe stehen, R3 und R4 jeweils unabhängig voneinander für Alkylgruppen mit 1 bis 4 Kohlenstoff-Atomen stehen, Y für eine NH-Gruppe und p für eine ganze Zahl von 2 bis 5 steht, und mindestens ein amphoteres Tensid, und ein Container eine Oxidationsmittel-zusammensetzung, enthaltend mindestens ein chemisches Oxidationsmittel, insbesondere Wasserstoffperoxid, enthält.

Wird eine besonders starke Aufhellwirkung durch Einsatz von Persulfatsalzen bzw. Peroxodisulfatsalzen gewünscht, ist es erfindungsgemäß bevorzugt, diese der erfindungsgemäßen Mehrkomponentenverpackungseinheit (Kit-of-Parts) in Form eines gegebenenfalls entstaubten Pulvers oder eines in Form gepressten Formkörpers als separat verpackte, zusätzliche Komponente beizufügen.

Bevorzugt enthält die Mehrkomponentenverpackungseinheit (Kit-of-Parts) zusätzlich eine Gebrauchsanleitung. Darüber hinaus kann es bevorzugt sein, wenn weiterhin eine Applikationshilfe, wie beispielsweise ein Kamm oder ein Pinsel, und/oder eine persönliche Schutzausrüstung, wie beispielsweise Einweg-Handschuhe dem Kit beigefügt ist.

Bezüglich weiterer bevorzugter Ausführungsformen der Mehrkomponentenverpackungseinheit (Kit-of-Parts) gilt mutatis mutandis das zu den erfindungsgemäßen Mitteln Gesagte.

Ein weiterer Gegenstand der Erfindung ist die Verwendung eines Mittels zum Färben keratinischer Fasern, insbesondere menschlicher Haare, enthaltend in einem kosmetischen Träger mindestens ein Oxidationsfarbstoffvorprodukt, dadurch gekennzeichnet, dass das Färbemittel zusätzlich mindestens ein nicht permanent ionisches Copolymer enthält, welches sich ableitet von mindestens einem kationisierbaren Monomer der allgemeinen Formel (I),

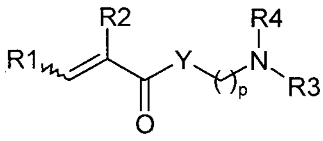

(I),

worin R1 und R2 jeweils unabhängig voneinander für Wasserstoff oder eine Methylgruppe stehen, R3 und R4 jeweils unabhängig voneinander für Alkylgruppen mit 1 bis 4 Kohlenstoff-Atomen stehen, Y für eine NH-Gruppe und p für eine ganze Zahl von 2 bis 5 steht,
zur Verbesserung des Farberhalts, insbesondere gegenüber Haarwäsche nach Farbveränderung menschlicher Haare.

Ein weiterer Gegenstand der Erfindung ist die Verwendung eines Mittels zum Färben keratinischer Fasern, insbesondere menschlicher Haare, enthaltend in einem kosmetischen Träger mindestens ein Oxidationsfarbstoffvorprodukt, dadurch gekennzeichnet, dass das Färbemittel zusätzlich mindestens ein nicht permanent ionisches Copolymer enthält, welches sich ableitet von mindestens einem kationisierbaren Monomer der allgemeinen Formel (I),

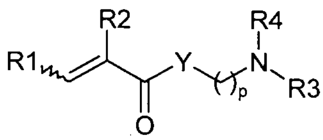

(I),

worin R1 und R2 jeweils unabhängig voneinander für Wasserstoff oder eine Methylgruppe stehen, R3 und R4 jeweils unabhängig voneinander für Alkylgruppen mit 1 bis 4 Kohlenstoff-Atomen stehen, Y für eine NH-Gruppe und p für eine ganze Zahl von 2 bis 5 steht,
zur Steigerung der Grauabdeckung.

Ein weiterer Gegenstand der Erfindung ist die Verwendung eines Mittels gemäß einem der Ansprüche 1 - 9 zur Verbesserung des Farberhalts.

Ein weiterer Gegenstand der Erfindung ist die Verwendung eines Mittels gemäß einem der Ansprüche 1 - 9 zur Steigerung der Grauabdeckung.

Bezüglich weiterer bevorzugter Ausführungsformen der erfindungsgemäßen Verwendungen gilt mutatis mutandis das zu den erfindungsgemäßen Mitteln Gesagte. Die nachfolgenden Beispiele sollen bevorzugte Ausführungsformen der Erfindung erläutern, ohne sie jedoch einzuschränken.

Beispiele

1.

Folgende erfindungsgemäß verwendete Färbecremes wurden hergestellt (Tabelle 1; Angaben in Gew.-%):

| **Rohstoff** | **E1** | **V1** |
|---|---|---|
| Hydrenol D | 8,10 | 8,10 |

(fortgesetzt)

| Rohstoff | E1 | V1 |
|---|---|---|
| Lorol C12-18, techn. | 2,80 | 2,80 |
| Texapon N 25 | 10,00 | 10,00 |
| Plantpon LGC Sorb | 5,00 | 5,00 |
| Eumulgin B 1 | 0,25 | 0,25 |
| Eumulgin B 2 | 0,25 | 0,25 |
| Iso-Stearinsäure | 2,00 | 2,00 |
| Edenor C14 | 0,50 | 0,50 |
| Produkt W37194 | 1,00 | 1,00 |
| Kaliumhydroxid | 1,20 | 1,20 |
| 1,2-Propandiol | 0,55 | 0,55 |
| Färbemischung* | 4,527 | 4,527 |
| Styleze CC10 | 2,00 | - |
| Natriumsulfit, wasserfrei, 96% | 0,50 | 0,50 |
| Ascorbinsäure | 0,40 | 0,40 |
| Ammoniumsulfat | 0,20 | 0,20 |
| Natriumsilikat | 0,50 | 0,50 |
| HEDP, 60% | 0,20 | 0,20 |
| Ammoniak, 25% | 3,00 | 3,00 |
| Parfum | qs | qs |
| Wasser | ad 100 | ad 100 |
| *Färbemischung, bestehend aus p-Amino-o-Kresol (0,73%), 2-Chlor-6-methyl-3-aminophenol (2,34%), p-Toluylendiaminsulfat (2,76%), 2-Methylresorcin (10,63%), 2,6-Dihdroxy-3,4-dimethylpyridin (14,58%), 4-Amino-3-methylphenol (3,53%), 2,4,5,6-Tetraaminopyrimidinsulfat (60,70%) und Resorcin (4,73%). | | |

Die Färbecremes E1 und V1 wurden jeweils vor der Anwendung im Gewichtsverhältnis 1:1 mit einer wie folgt zusammengesetzten Entwicklerdispersion (Tabelle 2) ausgemischt.

| Rohstoff | Gew.-% |
|---|---|
| Dipicolinsäure | 0,10 |
| Dinatriumpyrophosphat | 0,03 |
| HEDP, 60% | 1,5 |
| Texapon N 25 | 2,0 |
| Dow Corning DB 110 A | 0,067 |
| Aculyn 33A | 12,0 |
| Ammoniak, 25% | 0,62 |
| Wasserstoffperoxid (50 %) | 12,0 |
| Wasser | ad 100 |

Eingesetzte Rohstoffe:

| | |
|---|---|
| Hydrenol® D | $C_{16}$-$C_{18}$-Fettalkohol (INCI-Bezeichnung: Cetearyl Alcohol) (Cognis) |
| Lorol® C12-18, techn. | $C_{12}$-$C_{18}$-Fettalkohol (INCI-Bezeichnung: Coconut Alcohol) (Cognis) |
| Texapon® N 25 | $C_{12}$-$C_{14}$-Fettalkoholethersulfat (2 EO), Natriumsalz (27% Aktivstoff; INCI-Bezeichnung: Sodium Laureth Sulfate) (Cognis) |
| Plantapon® LGC Sorb | D-Glucose, verethert mit $C_{10}$-$C_{16}$-Fettalkohol; teilweise verethert mit Carboxymethyl, Natriumsalz (INCI-Bezeichnung: Lauryl Glucose Carboxylate, Lauryl Glucose) (Cognis) |
| Eumulgin® B1 | $C_{16}$-$C_{18}$-Fettalkohol, ethoxyliert (12 EO) (INCI-Bezeichnung: Ceteareth-12) (Cognis) |
| Eumulgin® B2 | $C_{16}$-$C_{18}$-Fettalkohol, ethoxyliert (20 EO) (INCI-Bezeichnung: Ceteareth-20) (Cognis) |
| Edenor® C14 | Tetradecansäure (INCI-Bezeichnung: Myristic Acid) (Cognis) |
| Produkt W37194® | Copolymer aus Trimethylammoniopropylacrylamid Chlorid / Acrylsäure Natriumsalz (INCI-Bezeichnung: Acrylamidopropyltrimonium Chloride Acrylates Copolymer) (Stockhausen) |
| Styleze® CC 10 | Copolymer aus Vinylpyrrolidinon / Dimethylaminopropylmethacrylamid (ca. 10% Aktivstoff, INCI-Bezeichnung: VP/DMAPA Acrylates Copolymer) (ISP) |
| Dow Corning® DB 110 A | nichtionische Siliconemulsion (ca. 10% Aktivstoff, INCI-Bezeichnung: Dimethicone) (Dow Corning) |
| Aculyn® 33A | Copoylmer aus Acrylsäure / Acrylsäureester (ca. 28% Aktivstoff, INCI-Bezeichnung: Acrylates Copolymer) (ISP) |

Für die Färbungen wurde das Haar von Probanden mit mittelblonder bis hellbrauner Ausgangshaarfarbe und einem Grauanteil von bis zu 30 % für eine Einwirkzeit von 30 min im Halbseitentest mit den anwendungsbereiten Färbemitteln aus E1 und V1 behandelt. Anschließend wurde das Haar mit einem handelsüblichen Shampoo gewaschen und mit einem Föhn getrocknet.
Visuelle Beurteilung ergab eine deutlich verbesserte Grauabdeckung bei der Verwendung des erfindungsgemäßen Färbemittels (E1) gegenüber dem Vergleichsfärbemittel (V1).
Ebenso ergab sich für die mit E1 erzielte Färbung nach 6maliger Haarwäsche mit einem handelsüblichen Shampoo ein signifikant verbesserter Farberhalt nach optischer Beurteilung.

2.

Folgende Färbecremes wurden hergestellt (Tabelle 3, Angaben in Gew.-%):

| | V2 | E2 | V3 | E3 |
|---|---|---|---|---|
| Hydrenol D | 7,0 | 7,0 | 7,0 | 7,0 |
| Lorol techn. | 4,2 | 4,2 | 4,2 | 4,2 |
| Eumulgin B2 | 0,35 | 0,35 | 0,35 | 0,35 |
| Eumulgin B3 | 0,35 | 0,35 | 0,35 | 0,35 |
| Mergital CS 50 A | 1,40 | 1,40 | 1,40 | 1,40 |
| Xanthan | 0,14 | 0,14 | 0,14 | 0,14 |
| 1,2-Propandiol | 0,7 | 0,7 | 0,7 | 0,7 |
| Merquat 281 | 3,0 | 3,0 | 3,0 | 3,0 |
| Amphoterge K-2 | 2,0 | 2,0 | 2,0 | 2,0 |
| Styleze CC10 | - | 2,0 | - | 2,0 |
| Styleze W 20 | - | - | 2,0 | 2,0 |
| p-Toluylendiaminsulfat | 1,429 | 1,429 | 1,429 | 1,429 |
| Resorcin | 0,745 | 0,745 | 0,745 | 0,745 |
| 4-Chlorresorcin | 0,44 | 0,44 | 0,44 | 0,44 |

(fortgesetzt)

| | V2 | E2 | V3 | E3 |
|---|---|---|---|---|
| 2-Amino-3-hydroxypyridin | 0,22 | 0,22 | 0,22 | 0,22 |
| Arginin | 1,0 | 1,0 | 1,0 | 1,0 |
| Ammoniumsulfat | 2,0 | 2,0 | 2,0 | 2,0 |
| Ascorbinsäure | 0,2 | 0,2 | 0,2 | 0,2 |
| Natriumsulfit, wasserfrei,96% | 0,2 | 0,2 | 0,2 | 0,2 |
| Natriumsilikat 40/42 | 0,5 | 0,5 | 0,5 | 0,5 |
| HEDP, 60% | 0,2 | 0,2 | 0,2 | 0,2 |
| Parfum | qs | qs | qs | qs |
| Monoethanolamin | 4,5 | 4,5 | 4,5 | 4,5 |
| Wasser | ad 100 | ad 100 | ad 100 | ad 100 |

Eingesetzte Rohstoffe:

| | |
|---|---|
| Eumulgin® B3 | $C_{16}$-$C_{18}$-Fettalkohol, ethoxyliert (30 EO) (INCI-Bezeichnung: Ceteareth-20) (Cognis) |
| Mergital® CS 50 | $C_{16}$-$C_{18}$-Fettalkohol, ethoxyliert (50 EO) (INCI-Bezeichnung: Ceteareth-50) (Cognis) |
| Merquat® 281 | Dimethyldiallylammoniumchlorid/Acrylsäure Copolymer (INCI-Bezeichnung: Polyquaternium-22) (Nalco) |
| Amphoterge® K-2 | (INCI-Bezeichnung: Disodium Cocoamphodipropionate) (Lonza) |
| Styleze® W 20 | Terpolymer aus N-Dodecyl-N,N-Dimethylammonio)propyl-methacrylamid Chlorid / Vinylpyrrolidinon / Dimethylaminopropylmethacrylamid (ca. 20% Aktivstoff, INCI-Bezeichnung: Polyquaternium-55) (ISP) |

Die Färbecremes E2 und E3 stellen erfindungsgemäße Zubereitungen dar, während die Mittel V2 und V3 Vergleichsrezepturen repräsentieren. Die Färbecremes E2, E3, V2 und V3 wurden jeweils vor der Anwendung im Gewichtsverhältnis 1:1 mit einer Entwicklerdispersion (s. 1.; Tabelle 2) ausgemischt. Es wurden Misch-pH-Werte von 9,3 bis 9,4 erreicht.

2a. Farberhalt

Für die Färbungen wurde auf Strähnen gebleichten Büffelbauchhaars von ca. 0,7 g Gewicht jeweils die 4-fache Menge der fertigen Anwendungsmischung appliziert. Nachdem die Strähnen für 10 Minuten bei 32 °C gefärbt wurden, wurden sie mit einem handelsüblichen Shampoo gewaschen und mit einem Föhn getrocknet.

Das gefärbte Haar wurde einem Cyclus aus 1 min Waschen mit einem handelsüblichen Shampoo und anschließendem Trocknen mit einem Föhn unterworfen. Dieser Cyclus wurde 6 mal wiederholt.

Für die Berechnung des Farberhalts wurden die ungefärbten Strähnen sowie die gefärbten Strähnen vor und nach den Waschcyclen mit Hilfe des Farbmessgerät der Firma Datacolor, Typ Spectraflash 450 vermessen. Der Farbabstand ΔE berechnet sich nach folgender Formel:

$$\Delta E_{1;2} = \sqrt{(L_1 - L_2)^2 + (a_1 - a_2)^2 + (b_1 - b_2)^2}$$

Hierbei steht der L-Wert für die Helligkeit (je geringer der L-Wert ist, desto größer ist die Farbintensität), während der a-Wert ein Maß für den Rotanteil einer Farbe ist (d. h. je größer der a-Wert ist, umso größer ist der Rotanteil). Der b-Wert ist ein Maß für den Blauanteil der Farbe, wobei der Blauanteil umso größer ist, je negativer der b-Wert ist.

Der Farberhalt FA wird üblicherweise als prozentualer Anteil der ursprünglichen Coloration im Vergleich zur gewaschenen Coloration angegeben und berechnet sich nach folgender Formel:

$$FA = 100 \cdot \left( \frac{\Delta E_{u;g}}{\Delta E_{u;c}} \right) = 100 \cdot \left( \frac{\sqrt{(L_u - L_g)^2 + (a_u - a_g)^2 + (b_u - b_g)^2}}{\sqrt{(L_u - L_c)^2 + (a_u - a_c)^2 + (b_u - b_c)^2}} \right)$$

Der Index u bezeichnet dabei uncoloriertes Haar, der Index c das colorierte, ungewaschene Haar, während der Index g, das colorierte, gewaschene Haar bezeichnet.

Folgende Werte für den Farberhalt wurden für die 4 Ausfärbungen ermittelt:

Tabelle 4.

| **Färbecreme** | **FA nach 6 Haarwäschen [%]** |
|---|---|
| *V2* | *90,1* |
| E2 | 97,3 |
| *V3* | *81,1* |
| E3 | 98,5 |

Es ist anhand der Werte aus Tabelle 4 offensichtlich, dass der Zusatz des erfindungsgemäßen nicht permanent ionischen Copolymers zur Färbecreme (E2 und E3) eine deutliche Steigerung des Farberhalts der damit erzielten Färbung bewirkt. Im Vergleich dazu zeigen die Ausfärbungen mit den Färbecremes (V2 und V3) ohne erfindungsgemäße Copolymere einen deutlich stärkeren Farbverlust nach dem Waschen.

2b. Grauabdeckung

Für die Färbungen wurde auf Strähnen von ca. 0,7 g Gewicht des unten genannten Haartyps jeweils die 4-fache Menge der fertigen Anwendungsmischung appliziert. Nachdem die Strähnen für 10 Minuten bei 32 °C gefärbt wurden, wurden sie mit einem handelsüblichen Shampoo gewaschen und mit einem Föhn getrocknet.

Für die Berechnung des Farbabstands zwischen ungefärbten und gefärbten Haaren wurden die gefärbten und ungefärbten Strähnen mit Hilfe des Farbmessgerät der Firma Datacolor, Typ Spectraflash 450 vermessen.

Zur Berechnung der Grauabdeckung wurde der Farbabstand zwischen gefärbtem, weißen Haar (BB) und gefärbtem Naturhaar (NH) in ein Verhältnis zum Farbabstand zwischen ungefärbtem, weißen Haar (NS) und gefärbtem Naturhaar (NH) gemäß folgender Formel (Grauab-deckungs-Index GAI in Prozent) gesetzt.

$$GAI = \left(1 - \frac{\Delta E_{BB;NH}}{\Delta E_{NS;NH}}\right) \cdot 100 = \left(1 - \frac{\sqrt{(L_{BB} - L_{NH})^2 + (a_{BB} - a_{NH})^2 + (b_{BB} - b_{NH})^2}}{\sqrt{(L_{NS} - L_{NH})^2 + (a_{NS} - a_{NH})^2 + (b_{NS} - b_{NH})^2}}\right) \cdot 100$$

Dabei wurde die Grauabdeckung für 3 unterschiedlich Naturhaarnuancen (mittelblond, mbl; hellbraun, hbr; dunkelbraun) untersucht. Je größer der GAI ist, desto besser ist das Grauabdeckungsvermögen der Färbemischung. Mit gemessenen ΔE-Werten ergeben sich für die oben beschriebenen Färbungen folgende Graudeckungs-Indices (Tabelle 5).

Tabelle 5.

| | **V2** | **V3** | **E2** | **E3** |
|---|---|---|---|---|
| **GAI** auf mittelblondem Haar (**mbl**) | 75,39 | 83,83 | **89,73** | **89,22** |
| **GAI** auf hellbraunem Haar (**hbr**) | 65,33 | 70,41 | **75,83** | **77,71** |
| **GAI** auf dunkelbraunem Haar (**dbr**) | 53,08 | 53,09 | **61,88** | **65,12** |

Aus Tabelle 5 ist ersichtlich, dass die erfindungsgemäßen Färbemischungen E2 und E3 gegenüber den Vergleichsfärbemischungen V2 und V3 ohne erfindungsgemäßes Copolymer auf allen drei Naturhaarnuancen (mbl, hbr, dbr) eine deutlich gesteigerte Grauabdeckung erzielen.

## Patentansprüche

1. Mittel zum Färben keratinischer Fasern, insbesondere menschlicher Haare, enthaltend in einem kosmetischen Träger mindestens ein Oxidationsfarbstoffvorprodukt und mindestens ein nicht permanent ionisches Copolymer enthält, welches sich ableitet von mindestens einem kationisierbaren Monomer der allgemeinen Formel (I),

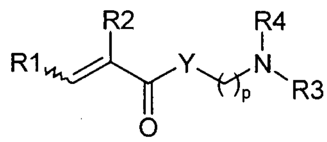

(I),

worin R1 und R2 jeweils unabhängig voneinander für Wasserstoff oder eine Methylgruppe stehen, R3 und R4 jeweils unabhängig voneinander für Alkylgruppen mit 1 bis 4 Kohlenstoff-Atomen stehen, Y für eine NH-Gruppe und p für eine ganze Zahl von 2 bis 5 steht,
**dadurch gekennzeichnet, dass** weiterhin mindestens ein amphoteres Tensid enthalten ist.

2. Mittel nach Anspruch 1, **dadurch gekennzeichnet, dass** das Copolymer sich mindestens von einem kationisierbaren Monomer der allgemeinen Formel (I) und/oder einem seiner mit einem Alkylierungsmittel kationisiertem Derivate ableitet, worin R2 für eine Methylgruppe und p für die Zahl 2 oder 3 steht.

3. Mittel nach einem der Ansprüche 1 oder 2, **dadurch gekennzeichnet, dass** das Copolymer sich mindestens von einem kationisierbaren Monomer der allgemeinen Formel (I) und/oder einem seiner mit einem Alkylierungsmittel kationisierten Derivate ableitet, worin R1 für Wasserstoff, R2, R3 und R4 jeweils für eine Methylgruppe, Y für eine NH-Gruppe und p für die Zahl 3 steht.

4. Mittel nach einem der Ansprüche 1 bis 3, **dadurch gekennzeichnet, dass** das Copolymer sich zusätzlich als weiteres Monomer von mindestens einem N-Vinyl-Lactam ableitet.

5. Mittel nach einem der Ansprüche 1 bis 4, **dadurch gekennzeichnet, dass** das Copolymer sich als weiteres Monomer von mindestens einem N-Vinyl-Lactam, ausgewählt aus N-Vinyl-pyrrolidin-2-on und N-Vinyl-azepan-2-on, ableitet.

6. Mittel nach einem der Ansprüche 1 bis 5, **dadurch gekennzeichnet, dass** das Copolymer sich mindestens von einem kationisierbaren Monomer der allgemeinen Formel (I), worin R1 für Wasserstoff, R2, R3 und R4 jeweils für eine Methylgruppe, Y für eine NH-Gruppe und p für die Zahl 3 steht, und mindestens N-Vinyl-pyrrolidin-2-on ableitet.

7. Mittel nach einem der Ansprüche 1 bis 6, **dadurch gekennzeichnet, dass** das Copolymer ein Molekulargewicht von 100,000 bis 500,000 g/mol, insbesondere von 250,000 bis 350,000 g/mol besitzt.

8. Mittel nach einem der Ansprüche 1 bis 7, **dadurch gekennzeichnet, dass** als amphoteres Tensid ein Tensid gemäß Formel (II) und/oder dessen physiologisch verträgliches Salz,

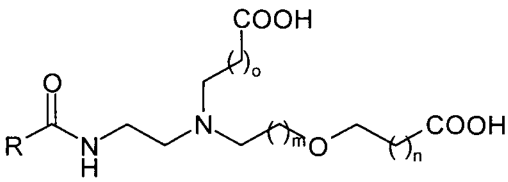

(II),

worin R für eine lineare oder verzweigte, gesättigte oder ungesättigte $C_{10}$-$C_{22}$-Alkylgruppe steht und m, n und o jeweils unabhängig voneinander für eine ganze Zahl 1, 2 oder 3 steht,
enthalten ist.

9. Mittel nach einem der Ansprüche 1 bis 8, **dadurch gekennzeichnet, dass** zusätzlich mindestens ein direktziehender Farbstoff enthalten ist.

**10.** Verfahren zum Färben menschlicher Haare, bei dem ein Mittel nach einem der Ansprüche 1 bis 9 auf das Haar aufgetragen wird, für eine Einwirkzeit von 2 bis 45 Minuten, bevorzugt von 5 bis 40 Minuten auf dem Haar belassen wird, und anschließend das Haar ausgespült wird.

**11.** Kit-of-Parts, enthaltend mindestens zwei getrennt konfektionierte Container, wobei ein Container eine Oxidationsfärbemischung gemäß einem Ansprüche 1 bis 9 enthält und ein Container eine Oxidationsmittelzusammensetzung, enthaltend mindestens ein chemisches Oxidationsmittel, insbesondere Wasserstoffperoxid, enthält.

**12.** Verwendung eines Mittels zum Färben keratinischer Fasern, insbesondere menschlicher Haare, enthaltend in einem kosmetischen Träger mindestens ein Oxidationsfarbstoffvorprodukt, **dadurch gekennzeichnet, dass** das Färbemittel zusätzlich mindestens ein nicht permanent ionisches Copolymer enthält, welches sich ableitet von mindestens einem kationisierbaren Monomer der allgemeinen Formel (I),

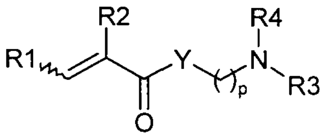

(I),

worin R1 und R2 jeweils unabhängig voneinander für Wasserstoff oder eine Methylgruppe stehen, R3 und R4 jeweils unabhängig voneinander für Alkylgruppen mit 1 bis 4 Kohlenstoff-Atomen stehen, Y für eine NH-Gruppe und p für eine ganze Zahl von 2 bis 5 steht,
zur Verbesserung des Farberhalts.

**13.** Verwendung eines Mittels gemäß einem der Ansprüche 1 - 9 zur Verbesserung des Farberhalts.

**14.** Verwendung eines Mittels zum Färben keratinischer Fasern, insbesondere menschlicher Haare, enthaltend in einem kosmetischen Träger mindestens ein Oxidationsfarbstoffvorprodukt, **dadurch gekennzeichnet, dass** das Färbemittel zusätzlich mindestens ein nicht permanent ionisches Copolymer enthält, welches sich ableitet von mindestens einem kationisierbaren Monomer der allgemeinen Formel (I),

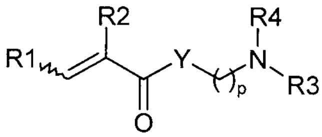

(I),

worin R1 und R2 jeweils unabhängig voneinander für Wasserstoff oder eine Methylgruppe stehen, R3 und R4 jeweils unabhängig voneinander für Alkylgruppen mit 1 bis 4 Kohlenstoff-Atomen stehen, Y für eine NH-Gruppe und p für eine ganze Zahl von 2 bis 5 steht,
zur Steigerung der Grauabdeckung.

**15.** Verwendung eines Mittels gemäß einem der Ansprüche 1 - 9 zur Steigerung der Grauabdeckung.

**Claims**

**1.** An agent for dyeing keratinic fibres, in particular human hair, containing in a cosmetic carrier at least one oxidation dye precursor and at least one non-permanent ionic copolymer derived from at least one cationisable monomer of general formula (I)

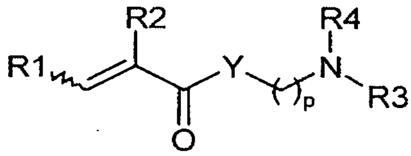

(I),

wherein R1 and R2 each represent independently from each other hydrogen or a methyl group, R3 and R4 each represent independently from each other alkyl groups with 1 to 4 carbon atoms, Y represents an NH group and p represents an integer of 2 to 5,
**characterised in that** further at least one amphoteric surfactant is contained.

2. The agent as claimed in claim 1, **characterised in that** the copolymer is derived at least from a cationisable monomer of general formula (I) and/or from any one of its derivatives cationised with an alkylating agent, wherein R2 represents a methyl group and p represents the number 2 or 3.

3. The agent as claimed in claim 1 or 2, **characterised in that** the copolymer is derived at least from a cationisable monomer of general formula (I) and/or from any one of its derivatives cationised with an alkylating agent, wherein R1 represents hydrogen, R2, R3 and R4 each represent a methyl group, Y represents an NH group and p represents the number 3.

4. The agent as claimed in any one of claims 1 to 3, **characterised in that** the copolymer is additionally derived as a further monomer from at least one N-vinyl-lactam.

5. The agent as claimed in any one of claims 1 to 4, **characterised in that** the copolymer is derived as a further monomer from at least one N-vinyl-lactam selected from N-vinyl-pyrrolidine-2-one and N-vinyl-azepane-2-one.

6. The agent as claimed in any one of claims 1 to 5, **characterised in that** the copolymer is derived at least from a cationisable monomer of general formula (I), wherein R1 represents hydrogen, R2, R3 and R4 each represent a methyl group, Y represents an NH group and p represents the number 3, and at least N-vinyl-pyrrolidine-2-one.

7. The agent as claimed in any one of claims 1 to 6, **characterised in that** the copolymer has a molecular weight of 100,000 to 500,000 g/mol, in particular of 250,000 to 350,000 g/mol.

8. The agent as claimed in any one of claims 1 to 7, **characterised in that** it contains as an amphoteric surfactant a surfactant according to formula (II) and/or the physiologically compatible salt thereof

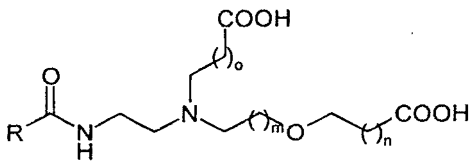

(II),

wherein R represents a linear or branched, saturated or unsaturated $C_{10}$-$C_{22}$ alkyl group and m, n and or each represent independently from each other an integer of 1, 2 or 3.

9. The agent as claimed in any one of claims 1 to 8, **characterised in that** it additionally contains at least one direct dye.

10. A method for dyeing human hair, wherein an agent as claimed in any one of claims 1 to 9 is applied to the hair, is left on the hair for an exposure time of 2 to 45 minutes, preferably of 5 to 40 minutes, and the hair is subsequently rinsed.

11. A kit of parts containing at least two separately prepared containers, wherein one container contains an oxidation dye mixture as claimed in any one of claims 1 to 9 and one container contains an oxidation agent composition containing at least one chemical oxidation agent, in particular hydrogen peroxide.

12. A use of an agent for dyeing keratinic fibres, in particular human hair, containing in a cosmetic carrier at least one oxidation dye precursor, **characterised in that** the dye additionally contains at least one non-permanent ionic

copolymer, which is derived from at least one cationisable monomer of general formula (I)

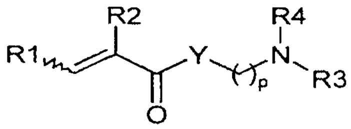

(I),

wherein R1 and R2 each represent independently from each other hydrogen or a methyl group, R3 and R4 each represent independently from each other alkyl groups with 1 to 4 carbon atoms, Y represents an NH group and p represents an integer of 2 to 5,
for enhancing colour retention.

13. The use of an agent as claimed in any one of claims 1 to 9 for enhancing colour retention.

14. The use of an agent for dyeing keratinic fibres, in particular human hair, containing in a cosmetic carrier at least one oxidation dye precursor, **characterised in that** the dye additionally contains at least one non-permanent ionic copolymer derived from at least one cationisable monomer of general formula (I)

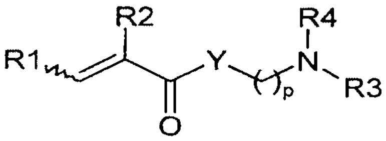

(I),

wherein R1 and R2 each represent independently from each other hydrogen or a methyl group, R3 and R4 each represent independently from each other alkyl groups with 1 to 4 carbon atoms, Y represents an NH group and p represents an integer of 2 to 5,
for enhancing grey coverage.

15. The use of an agent as claimed in any one of claims 1 to 9 for enhancing grey coverage.

## Revendications

1. Composition de coloration de fibres kératiniques, en particulier de cheveux humains, contenant dans un support cosmétique au moins un précurseur de colorant d'oxydation et au moins un copolymère non ionique permanent qui est dérivé d'au moins un monomère cationisable de la formule générale (I),

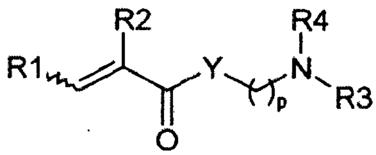

(I),

dans laquelle R1 et R2 représentent chacun indépendamment un atome d'hydrogène ou un groupe méthyle, R3 et R4 représentent chacun indépendamment des groupes alkyles comportant 1 à 4 atomes de carbone, Y représente un groupe NH et p est un nombre entier de 2 à 5, **caractérisée en ce qu'**elle contient en outre au moins un tensioactif amphotère.

2. Composition selon la revendication 1, **caractérisée en ce que** le copolymère est dérivé d'au moins un monomère cationisable de la formule générale (I) et/ou de l'un de ses dérivés cationisés avec un agent d'alkylation, formule dans laquelle R2 représente un groupe méthyle et p est le nombre 2 ou 3.

3. Composition selon l'une des revendications 1 ou 2, **caractérisée en ce que** le copolymère est dérivé d'au moins un monomère cationisable de la formule générale (I) et/ou d'un de ses dérivés cationisés avec un agent d'alkylation,

formule dans laquelle R1 représente un atome d'hydrogène, R2, R3 et R4 représente chacun un groupe méthyle, Y représente un groupe NH, et p est le nombre 3.

4. Composition selon l'une des revendications 1 à 3, **caractérisée en ce que** le copolymère est dérivé en outre, sous la forme d'un autre monomère, d'au moins un N-vinyl-lactame.

5. Composition selon l'une des revendications 1 à 4, **caractérisée en ce que** le copolymère est dérivé, sous la forme d'un autre monomère, d'au moins un N-vinyl lactame choisi parmi N-vinyl-pyrrolidine-2-one et N-vinyl-azépan-2-one.

6. Composition selon l'une des revendications 1 à 5, **caractérisée en ce que** le copolymère est dérivé d'au moins un monomère cationisable de la formule générale (I) dans laquelle R1 représente un atome d'hydrogène, R2, R3 et R4 représentent chacun un groupe méthyle, Y représente un groupe NH et p est le nombre 3, et dérive au moins de la N-vinyl-pyrrolidine-2-one.

7. Composition selon l'une des revendications 1 à 6, **caractérisée en ce que** le copolymère a un poids moléculaire de 100 000 à 500 000 g/mol, en particulier de 250 000 à 350 000 g/mol.

8. Composition selon l'une des revendications 1 à 7, **caractérisée en ce que** le tensioactif amphotère qu'elle contient est un tensioactif de la formule (II) et/ou un de ses sels physiologiquement acceptables,

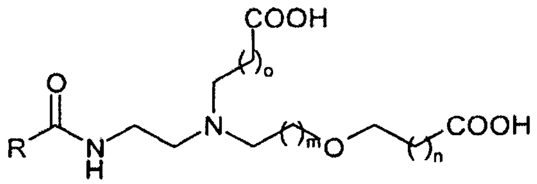

(II),

formule dans laquelle R représente un groupe alkyle en $C_{10}$-$C_{22}$ linéaire ou ramifié, saturé ou insaturé, et m, n et o sont chacun indépendamment un nombre entier égal à 1, 2 ou 3.

9. Composition selon l'une des revendications 1 à 8, **caractérisée en ce qu'**elle contient en plus au moins un colorant direct.

10. Procédé de teinture de cheveux humains, dans lequel l'on applique sur les cheveux une composition selon l'une quelconque des revendications 1 à 9, on la maintient pendant un temps de réaction de 2 à 45 minutes, de préférence de 5 à 40 minutes sur les cheveux, puis on rince les cheveux.

11. Kit d'éléments, comprenant au moins deux récipients fabriqués séparément, un récipient contenant un mélange de colorants d'oxydation selon l'une des revendications 1 à 9 et un récipient contenant une composition d'agent d'oxydation contenant au moins un agent d'oxydation chimique, en particulier du peroxyde d'hydrogène.

12. Utilisation d'un agent de coloration de fibres kératiniques, en particulièrement des cheveux humains, contenant dans un support cosmétique au moins un précurseur de colorant d'oxydation, **caractérisée en ce que** le colorant contient en outre au moins un copolymère ionique non permanent qui est dérivé d'au moins un monomère cationisable de la formule générale (I),

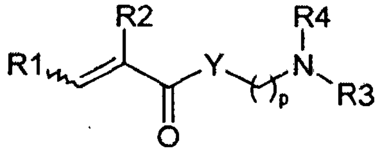

(I),

dans laquelle R1 et R2 représentent chacun indépendamment un atome d'hydrogène ou un groupe méthyle, R3 et R4 représentent chacun indépendamment des groupes alkyles comportant 1 à 4 atomes de carbone, Y représente un groupe NH et p est un nombre entier de 2 à 5, pour améliorer la préservation de la couleur.

**13.** Utilisation d'une composition selon l'une des revendications 1 à 9 pour améliorer la préservation de la couleur.

**14.** Utilisation d'une composition de coloration de fibres kératiniques, en particulièrement des cheveux humains, contenant dans un support cosmétique au moins un précurseur de colorant d'oxydation, **caractérisée en ce que** le colorant contient en outre au moins un copolymère ionique non permanent dérivé d'au moins un monomère cationisable de la formule générale (I)

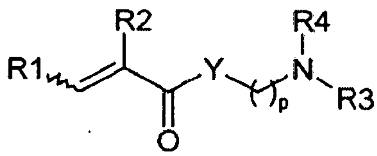

(I),

dans laquelle R1 et R2 représentent chacun indépendamment un atome d'hydrogène ou un groupe méthyle, R3 et R4 représentant chacun indépendamment des groupes alkyles comportant 1 à 4 atomes de carbone, Y représente un groupe NH et p est un nombre entier de 2 à 5, pour augmenter le camouflage des cheveux gris.

**15.** Utilisation d'une composition selon l'une des revendications 1 à 9 pour augmenter le camouflage des cheveux gris.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- FR 2820032 A1


### In der Beschreibung aufgeführte Nicht-Patentliteratur


- **KH. SCHRADER.** Grundlagen und Rezepturen der Kosmetika. Hüthig Buch Verlag, 1969